# EUROPEAN PATENT SPECIFICATION

(11) **EP 3 082 708 B1**
(45) Date of publication and mention of the grant of the patent: **03.04.2019**
(21) Application number: 14872578.1
(22) Date of filing: 12.12.2014
(51) Int. Cl.: A61K 9/00, A61J 3/02, A61K 31/40, B02C 17/20, A61K 31/573, A61K 9/16

(54) **MEDIA MILLING PROCESS FOR THE MANUFACTURE OF ACTIVE PHARMACEUTICAL INGREDIENTS IN PROPELLANTS**
MEDIENMAHLVERFAHREN ZUR HERSTELLUNG VON PHARMAZEUTISCHEN WIRKSTOFFEN IN TREIBMITTELN
PROCÉDÉ DE BROYAGE DE MILIEUX POUR LA FABRICATION DE COMPOSANTS PHARMACEUTIQUES ACTIFS DANS DES PROPULSEURS

(30) Priority: 17.12.2013 US 201361916960 P
(43) Date of publication of application: 26.10.2016
(73) Proprietor: Merck Sharp & Dohme Corp., Rahway, NJ 07065-0907 (US)
(72) Inventor: BHARATWAJ, Balaji, Rahway, New Jersey 07065-0907 (US); CHAMARTHY, Sai Prasanth, Rahway, New Jersey 07065-0907 (US); OREKIE, Chinedu, G., Rahway, New Jersey 07065-0907 (US); SON, Yoen-Ju, Rahway, New Jersey 07065-0907 (US)
(74) Representative: Jaap, David Robert
(86) International application number: PCT/US2014/069871
(87) International publication number: WO 2015/094927

(56) References cited:
- WO-A1-2013/066735
- WO-A1-2013/144655
- US-A1- 2003 068 280
- US-A1- 2005 287 077
- US-A1- 2012 301 528
- JUAN G. OSORIO ET AL: "Evaluation of resonant acoustic mixing performance", POWDER TECHNOLOGY, vol. 278, 1 July 2015 (2015-07-01), pages 46-56, XP055213400, ISSN: 0032-5910, DOI: 10.1016/j.powtec.2015.02.033

## Description

### BACKGROUND OF THE INVENTION

Particle size is a critical attribute to any pharmaceutical dosage form. For instance, effective delivery of drugs to the deep lung requires a stringent particle size range of about 0.5 to about 5 µm. In the case of tablets, formulation performance and dissolution kinetics can be significantly improved with particle size reduction of the active pharmaceutical ingredient (API). This is particularly important with biopharmaceutical classification system (BCS) class 2 compounds that have bioavailability issues associated with limited solubility. To achieve the desired API size range, milling is typically employed in the pharmaceutical industry to reduce the particle size of the (API). However, employing conventional milling approaches (for example, jet mill, conical mill, hammer mill and ball mill) to obtain particles of the stipulated size range may often time result in generation of an undesirable change in the solid state of the milled product (crystalline to amorphous). The presence of any amorphous content in the milled product could potentially impact the stability of the resulting formulation during manufacturing and storage as the amorphous form is inherently unstable. This is also true when the API is milled in propellants. WO 2013/144655 discloses a process using an acoustic mixing device, comprising the steps of mixing a particulate API, a particulate additive, and a propellant.

In view of the foregoing, there is a need for improving the process by which API is milled in propellants to reduce or negate the formation of amorphous product.

### SUMMARY OF THE INVENTION

We disclose a universal media milling technique (process) for the manufacture of respirable size range API and/or excipients (product) in propellants. With this process, we were able to obtain a milled product with substantially less amorphous content. Consequently, the resulting product was highly stabile and able to retain its original size without exhibiting appreciable particle growth for as long as 4 weeks when subjected to accelerated conditions of stability testing. This is particularly important in the case of hygroscopic or moisture sensitive APIs that have the propensity to rapidly grow under elevated conditions of humidity. The relevance of this new process was underscored when applied to the milling of glycopyrrolate bromide (GP), a long acting muscarinic agent (LAMA) - a compound that is highly unstable when exposed to moisture and is typically jet milled to respirable size. When exposed to accelerated conditions of humidity (75%) and temperature (40°C) jet milled samples of GP double their X50 (median diameter of the particle size distribution) within 6 hours and the particle-size distribution (PSD) was immeasurable after exposure beyond 24h due to significant particle growth. However, GP milled using the instant process, was stable at accelerated conditions of stability for a period of over 4 weeks.

### BRIEF DESCRIPTION OF THE DRAWINGS

FIGURE 1: Process diagram describing the LabRAM-based *in situ* hydrofluoroalkane-media (HFA-media) milling (HFA-LabRAM) method.
FIGURE 2: (A) PSD profile of unmilled GP before and after HFA-LabRAM milling as determined by SYMPATEC particle size analyzer (B) Differential scanning calorimetry (DSC) thermograms of unmilled and HFA-LabRAM milled GP.
FIGURE 3: (A) PSD profile of unmilled β-lactose before and after HFA-LabRAM milling (B) DSC thermograms of unmilled and HFA-LabRAM milled β-lactose.
FIGURE 4: Variation in PSD of HFA-LabRAM milled GP after exposure to (A) 75% Relative Humidity (RH) and 25°C (achieved by placing sample in RH chamber equilibrated with Bovada 75% RH packs) and (B) 75% RH and 40°C. Data from jet milled GP is also included in (B). Note that no PSD measurement could be done after the 6h time (for the jet milled GP) point owing to particles growing to size beyond the measurement capabilities of the SYMPATEC device. 10µm data shown at 24h is due to visual observation due to substantial API growth at the time of measurement.

### DETAILED DESCRIPTION OF THE INVENTION

The invention disclosed herein is a novel media milling process according to claim 1, performed in an atmosphere of propellant(s) utilizing a resonant acoustic mixing (RAM) device. The process is utilized to reduce the particle size of API (optionally including excipients) to a respirable size range while ensuring the retention of the crystallinity of the milled API. This process can be utilized for any API and/or excipient that have little or no solubility in propellants. More specifically, hygroscopic or moisture sensitive API(s) that have a propensity to grow under accelerated conditions of humidity are preferred API(s) to use for this process. Additionally, API (product) that requires special storage and manufacturing requirements (low RH storage and/or refrigeration etc.) would greatly benefit from this milling process.

Acoustic mixing devices, for example, the Resodyn™ acoustic mixer are commercially available. This technology has been described, for example, in U.S. Patent 7,188,993 to Howe et al., and employs linear displacement to introduce a standing linear wave into a medium, for example, gas, liquid or solid, residing within a container affixed to the device. Preparation of admixtures comprising energetic or shock-sensitive materials has been described using acoustic mixing, for example, in published U.S. Patent Application 2010/0294113 (McPherson). Suspension of pre-formed nanoparticulate materials in an aqueous medium has also been described, for example, the dispersion of silver nanoparticles of 20 nm - 30 nm in water using an acoustic mixing device (Resodyn™ marketing literature). Acoustic mixing devices have also been used in process to prepare nano-suspensions, for example, in WO2013/066735.

In an embodiment of the instant invention is disclosed a milling process comprising (A) adding an API, in the presence or absence of excipients and/or additives, and beads to a container; (B) sealing the container and filling with propellant media; and (C) mixing the container with an acoustic mixing device.

In another embodiment the propellant media is selected from HFA227 or HFA134a or a combination of both in varying proportions.

In another embodiment the acoustic mixing device is the Resodyn™ LabRAM™ acoustic mixer.

In another embodiment the process does not comprise excipients or additives.

The beads are of a particular size range from .05 to 5mm. In another embodiment the bead size is from about .1 to about 2mm.

In another embodiment the ratio of bead size to API (including or excluding any excipients and/or additives) is from about 40:1, or about 20:1, or about 15:1, or about 10:1 or about 5:1.

In another embodiment the acoustic mixing devise emits acoustic energy in about 10 to about 100 Hertz frequency.

In another embodiment the acoustic mixing devise emits acoustic energy with a force of from about 10 G to about 100 G (wherein G is the force of gravity).

In another embodiment the API is selected from ICS (inhaled corticosteroids), inhaled inhibitors of SYK (iSYK) and JAK (iJAK) and LAMAs, LABAs, MABAs and SABAs, or combinations thereof.

In another embodiment the API is selected from mometasone furoate and GP.

In another embodiment the API is GP.

In another embodiment mixing occurs over a specified time range from about 5 minutes to about 300 minutes. In another embodiment mixing occurs over a specified time range from about 30 minutes to about 200 minutes. In another embodiment mixing occurs over a specified time range from about 60 minutes to about 120 minutes.

In another embodiment of the instant invention is a product obtained by the milling process disclosed herein.

"Additives" means agents that could be added in small quantities to improve and stabilize the milling process. Additives include but are not limited to surfactants, stabilizers, emulsifiers including, but not limited to oleic acid, polyethylene glycol (PEG), sorbitan trioleate, polysorbates, pluronic range of surfactants, polyvinylpyrrolidone (PVP), lactose, polysaccharides (cellulose, arabinose, galactomannan), chitosan, sugar alcohols (mannitol, sorbitol, xylitol) and cyclodextrins.

"API" means an active pharmaceutical ingredient. Examples of APIs include corticosteroids (ICS) such mometasone furoate; beclomethasone dipropionate; budesonide; fluticasone; dexamethasone; flunisolide; triamcinolone; (22R)-6.alpha., 9.alpha.-difluoro-11.beta., 21-dihydroxy-16.alpha., 17.alpha.-propylmethylenedioxy-4-pregnen-3,20-dione, tipredane, GSK685698, GSK799943 or a pharmaceutically acceptable salt or hydrate of any of the above, or a combination of two or more of the above,; long acting beta agonists (LABAs) such as formoterol, salmeterol, bambuterol, indacaterol, vilanterol, carmoterol, TA-2005, or a pharmaceutically acceptable salt or hydrate of any of the above, or a combination of two or more of the above. Suitable short acting beta agonist include albuterol, terbutaline sulfate, bitolterol mesylate, levalbuterol, metaproterenol sulfate, pirbuterol acetate or a pharmaceutically acceptable salt or hydrate of any of the above, or a combination of two or more of the above.long acting muscarinic agents (LAMAs) such as (R)-3-[2-hydroxy-2,2-(dithien-2-yl)acetoxy]-1-1[2-(phenyl)ethyl]-1-azoniabicyclo[2.2.2]octane, glycopyrrolate, ipratropium bromide, oxitropium bromide, atropine methyl nitrate, atropine sulfate, ipratropium, belladonna extract, scopolamine, scopolamine methobromide, methscopolamine, homatropine methobromide, hyoscyamine, isopriopramide, orphenadrine, benzalkonium chloride, tiotropium bromide, GSK202405, an individual isomer of any of the above or a pharmaceutically acceptable salt or hydrate of any of the above, or a combination of two or more of the above; suitable phosphodiesterase IV inhibitors include cilomilast, roflumilast, tetomilast, 1-[[5-(1(S)-aminoethyl)-2-[8-methoxy-2-(trifluoromethyl)-5-quinolinyl]-4-oxazolyl]carbonyl]-4(R)-[(cyclopropylcarbonyl)amino]-L-proline, ethyl ester or a pharmaceutically acceptable salt or hydrate of any of the above, or a combination of two or more of the above ; tyrosine kinase inhibitors and Bifunctional Muscarinic Antagonist-Beta2 Agonist (MABAs) such as GSK961081 or combinations of above APIs thereof. API can also include biologics (proteins, peptides, monoclonal antibodies (mABs)). Other APIs that are suitable to be size reduced via this approach are APIs for central nervous disorders including, but not limited to, Suvorexant, dihydroergotamine (DHE) for pain and combinations thereof. Particularly preferred API include those API that are hygroscopic. Any API not soluble or sparingly soluble in propellant media can be milled using the instant process. API can also include biologics (proteins, peptides, monoclonal antibodies (mABs)). Particularly preferred API include those API that are moisture and temperature sensitive.

"Acoustic energy" means the linear or spherical energy propagation through a tangible medium which is within the frequency range of 10 hertz to 20,000 hertz.

"Acoustic mixing device" means a device capable of supplying acoustic energy. Examples of acoustic mixing devices are RAM (Resodyn acoustic mixers) devices. A preferred acoustic mixing device is the Resodyn™ LabRAM™ acoustic mixer

"Beads" means agents of various diameters that facilitate grinding and size reduction of API and any additives and/or excipients subjected to milling. The beads consist in various ceramic grinding media like zirconia and Yttria-stabilized zirconia (YTZ).

"Container" means an enclosed vessel in which the milling is undertaken. Typical containers used should be able to withstand pressures of approximately 50 to about 100 pounds per square inch (psi). Preferred containers include but are not limited to glass bottles, quartz vessels and stainless steel containers.

"Excipients" means inactive ingredients formulated along with API in order to bulk up the drug product and/or lend stability to the final formulation. Excipients include but are not limited to surfactants, stabilizers, emulsifiers including, but not limited to oleic acid, polyethylene glycol (PEG), sorbitan trioleate, polysorbates, pluronic range of surfactants, polyvinylpyrrolidone (PVP), lactose, polysaccharides (cellulose, arabinose, galactomannan), chitosan, sugar alcohols (mannitol, sorbitol, xylitol) and cyclodextrins.

"Propellant media" means chloroflurocarbons (CFCs) and hydrofluoroalkanes (HFAs). CFCs are selected from CFC11, CFC12 and CFC14. HFAs, also known as hydrofluorocarbons (HFCs) are selected from HFA227 or HFA134a or a combination of both at varying ratios. Preferred propellants are HFAs.

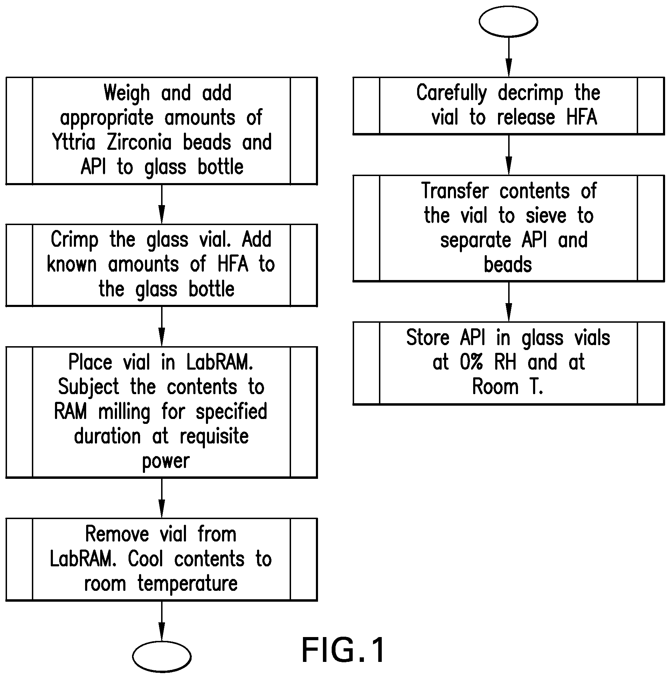

In a typical RAM based media milling process (see Figure 1), API is added to a glass bottle along with Yttria zirconia (YTZ, diameters ranging from about 0.1 to about 2mm) beads. Preferred bead sizes are from .05 to 5mm. Another preferred bead size is from about .1 to about 2mm. Other components that can be added to the API-bead mixture include stabilizers and surfactants that can augment the milling process. The ratio of the beads to that of the formulation components can be varied to suit the needs of particle size requirements. Particularly preferred ratios of beads to formulation components are from 40:1, 20:1, 15:1, 10:1 and 5:1. The bottle is crimp sealed and filled with appropriate amounts of either HFA227 or HFA134a (or a combination of both at varying ratios). The bottle is then loaded onto a RAM device and milled for a specified duration of time and at a specified power. Milling times include between about 5 minutes to about 300 minutes. Preferred milling times are from about 30 minutes to about 200 minutes and 60 minutes to about 120 minutes. Power includes about 10% to about 100% power. Preferred power range is about 40% to about 100%. Further, preferred power range is about 75% to about 100%. In most cases, the process can proceed to completion without stoppage. However, because of the ease and simplicity of the process, the process flow can be readily altered by stopping the media milling at intermittent time points for a known period of time before restarting the process again. This may be performed to cool the contents of the bottle at regular intervals. After completion, the bottle is allowed to revert to room temperature (if needed) to rapidly vent the HFA. After the process is finished the contents of the bottle (API, any excipients and/or additives, and the beads) are then added to a sieve of appropriate mesh size to separate the beads and the API. The milled API is collected and stored at 0% RH (in a nitrogen glove box) for further use.

The particle size of the product obtained by this process can vary to between about 0.5 to about 10µm. Typically API is reduced to the size range of about 0.5 to about 5.5µm. More specifically, the API can be reduced to a size range of about 0.5 to about 1.0µm, or about 1.0 to about 3.0µm, or about 2 to about 5.5µm. Duration of use and power required by the resonant acoustic mixing device is contingent upon the requisite final particle size of the product. Other parameters that can influence the final particle size of the product include bead size, bead ratio (with respect to API content) and total solids (API plus any excipients) to HFA propellant ratio. This process is applicable to a wide range of API and not limited to medications delivered via the inhaled route. Any API and/or excipient not soluble in HFA propellants and requiring a size reduction can be milled using this approach - separately or in combination.

### EXAMPLES

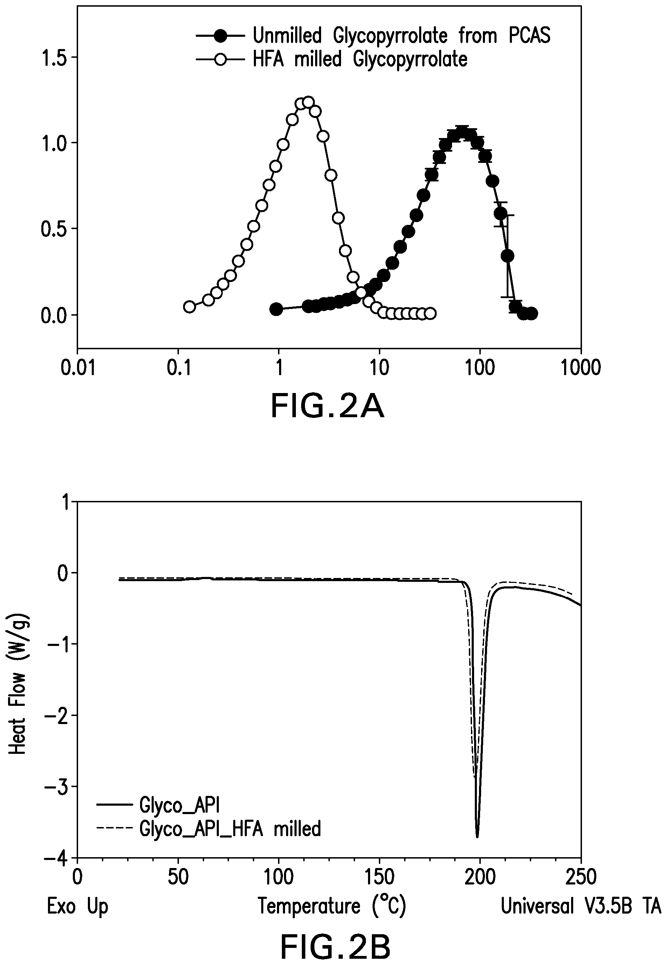

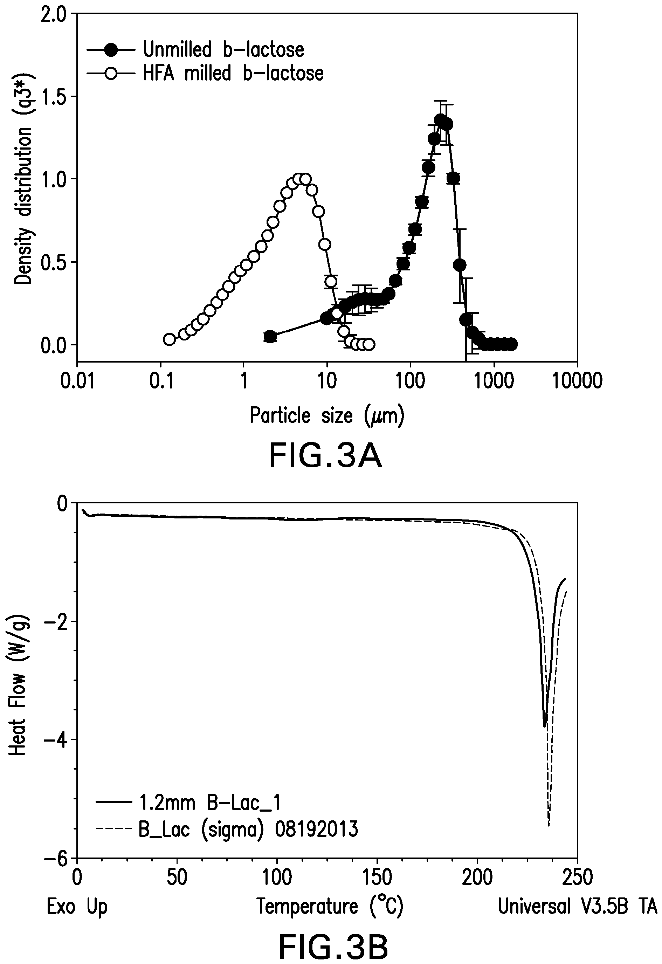

With respect to the examples below, the milled API was subject to DSC and the thermograms compared to that of the neat unmilled API. The data from the thermograms clearly indicate that the RAM based HFA-milled API maintains its crystalline structure (Figure 2B). It was also confirmed that the RAM milling process minimized the undesirable amorphous material generation during milling. In the case of lactose, for instance, as shown in Figure 3B, there is no glass transition on the DSC thermogram of the HFA-LabRAM milled material, indicating the crystallinity of the API was not influenced by the milling process.

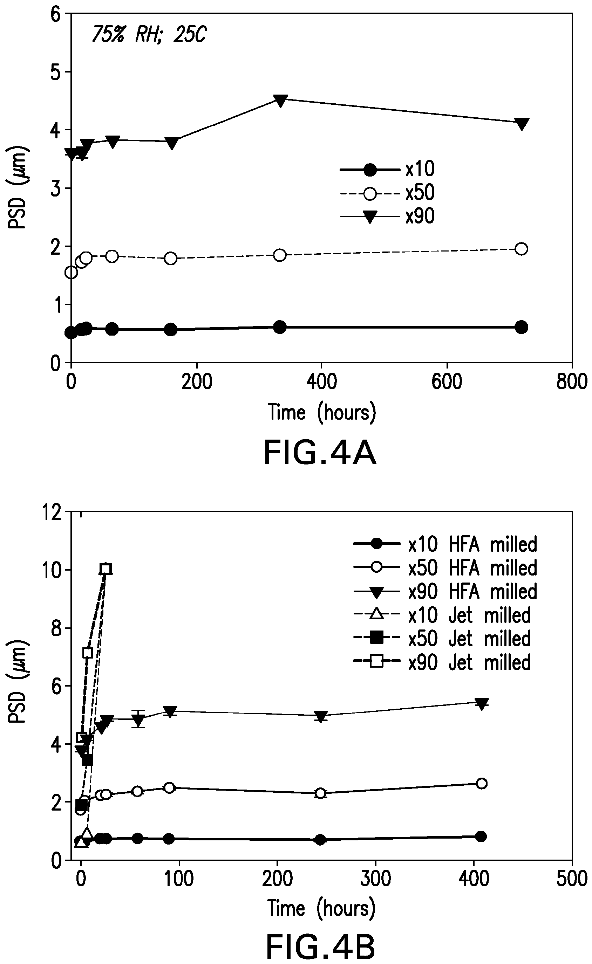

Functionally the presence of amorphous fraction in a milled API is often manifested as a change in PSD upon exposure to moisture and temperature. Some API are more sensitive than others. Milled GP is very sensitive to this phenomenon and exhibits significant growth (Figure 4B). In order to challenge our system the HFA-LabRAM milled product was subjected to accelerated stability studies at 75% RH and two different temperatures 25°C and 40°C. The particle size of the API stored in the humidity chamber was measured at regular intervals using a SYMPATEC particle size analyzer. Storage of the API at 75% RH and 25°C resulted in an increase in particle size (X50) from 1.54 to 1.78 µm (Figure 4A) within a span of 24h. For the API stored at 75% RH and 40°C, an increase in PSD of 0.5 µm was observed within 20h. Conversely, jet milled API grew to double its size within a period of 6 hours at 75% RH and 40°C conditions of stability and a 100 fold increase in the size of the API was recorded within 24h (Figure 4B).

The procedure requires the use of HFA (either HFA227 or HFA134a or a combination of both) which are staple propellants used in MDI formulations. YTZ beads (of varying sizes ranging from about 0.05 to about 5mm), is typically used as the grinding media and is added to the propellant. The HFA amount and the solids content in the propellant can be varied to suit the requirements of the final product. The process described herein is typically applicable for inhalation of medicaments (oral and nasal). However, API milled using this approach can be repurposed to suit other modes of administration

The following are illustrative embodiments of the invention not limiting the scope in any way.

### Example 1: Glycopyrrolate (GP)

**Table 1. Compendium of HFA227-milled GP at various processing conditions.**

| **PSD before HFA Milling** | | **PSD after HFA Milling** | | **Power and time** | **API:Bead ratio (w/w)** |
|---|---|---|---|---|---|
| **X50** | **X90** | **X50** | **X90** | | |
| 52.77±1.54 | 134.7±9.9 | 3.49±0.02 | 15.93±0.14 | 80% power; 60 mins | 20 |
| 52.77±1.55 | 134.7±9.10 | 2.62±.01 | 9.83±0.01 | 80% power; 105 mins | 20 |
| 52.77±1.54 | 134.7±9.9 | 1.53±0.06 | 3.67±0.06 | 80%power; 90 min; 5 min stop @45 | 10 |
| 52.77±1.55 | 134.7±9.10 | 1.7 | 4.53±0.21 | 80%power; 86 min | 20 |
| 52.77+1.56 | 134.7±9.11 | 1.7 | 3.87±0.12 | 80%power; 90 min; 5 min stop @45 | 10 |
| 52.77±1.57 | 134.7±9.12 | 1.37±0.13 | 3.67±0.03 | 80%power; 90 min; 5 min stop @45; 10 min stop; 90% power for additional 3 mins | 20 |

In one embodiment of the invention, GP was subjected to HFA-based media milling using LabRAM. GP is a highly hygroscopic API whose instability under accelerated conditions of temperature and humidity (40°C and 75% RH) has been widely documented. Typically, size reduction of GP is accomplished using conventional techniques like jet milling which result in generation of sizeable amorphous content that has led to the aforementioned instability.

GP was milled utilizing LabRAM in both HFA134a and HFA227. The milling was accomplished at different parameters of solids ratio, milling duration and power. YTZ beads (0.8µm) were utilized. In all cases reduction in X50 of the API was achieved from a size of ca. 51 µm to less than 3µm with no change in the polymorphic state of the API. In one particular embodiment, increasing the solids ratio in HFA134a resulted in the API size reduced to a respirable size range with an average X50 of 1.5µm. Unexpectedly, the HFA-LabRAM milled GP, when subjected to accelerated conditions of stability, did not exhibit noticeable particle growth for up to a month while the jet milled GP grew within 6 hours of exposure to accelerated conditions of stability (Figure 4A).

### Example 2: Lactose

Lactose is an excipient that is FDA approved and is utilized as a carrier in dry powder inhalers. In one variation of the milling study, β-lactose (anhydrous) purchased from sigma-aldrich was subjected to HFA (HFA227) based media milling using LabRAM at a preset power of 80% and for a duration of 70 minutes. The ratio of YTZ beads (0.8µm) to β-lactose was maintained at 20:1. Particle size (X50) of β-lactose prior to HFA-based RAM milling was 153.6±7.4µm. Upon subjecting the unmilled lactose to RAM-milling under the aforementioned conditions, the particle size (X50) of β-lactose was 3.47±0.01 µm.

In another embodiment of the study, the same β-lactose was subject to milling in HFA134a at a bead to lactose ratio of 40:1 for a 60 minute duration. The particle size (X50) of the recovered product was 2.3µm. In both cases, DSC results indicated that the final product was crystalline.

Lactose monohydrate of different grades (ML001 and SV003) was purchased from DFE pharma and subject to milling using LabRAM in both HFA227 and HFA134a media. Two different ratios of bead to lactose were evaluated at varying conditions of milling. Upon RAM-milling with HFA227, X50 of lactose was reduced to a size less than 3µm while under similar conditions, HFA134a RAM-milled lactose was reduced to an X50 of 1.9µm.

### Example 3: Cyclodextrin (Captisol)

Cyclodextrin is a widely utilized pharmaceutical excipient used as a solubility enhancer and complexing agent in several formulations. Captisol subjected to HFA based LabRAM milling resulted in a size reduction of the excipient to 1.5µm.

## Claims

1. A milling process comprising (A) adding an API, in the presence or absence of excipients and/or additives, and beads to a container; (B) sealing the container and filling with propellant media; and (C) mixing the container with an acoustic mixing device, wherein the beads are ceramic grinding media having a particular size range from .05 to 5 mm.

2. The process of Claim 1 wherein the propellant media is selected from HFA227 or HFA134a or a combination of both in varying proportions.

3. The process of claim 1 wherein the bead size is from .1 to 2 mm.

4. The process of Claim 1 wherein the ratio of bead size to API is from 40:1, or 20:1, or 15:1, or 10:1 or 5:1.

5. The process of Claim 1 wherein the API is selected from inhaled corticosteroids (ICS), inhaled inhibitors of SYK (iSYK) and JAK (iJAK), long acting muscarinic agents (LAMAs), long acting β-agonists (LABAs), bifunctional muscarinic antagonist β-2 agonists (MABAs) and short acting β-agonists (SABAs), or combinations thereof.

6. The process of Claim 5 wherein the API is selected from mometasone furoate and glycopyrrolate (GP).

7. The process of Claim 6 wherein the API is GP (glycopyrrolate).

8. The process of Claim 1 wherein the mixing occurs over a specified time range from 5 minutes to 300 minutes.

9. A product obtained by the milling process of Claim 1.

## Patentansprüche

1. Ein Mahlverfahren, umfassend (A) das Hinzufügen eines API, in Gegenwart oder Abwesenheit von Hilfsstoffen und/oder Additiven, und Perlen zu einem Behälter; (B) das Verschließen des Behälters und Befüllen mit Treibmittel; und (C) das Mischen des Behälters mittels einer Akkustikmischvorrichtung, wobei die Perlen keramische Mahlkörper mit einem speziellen Größenbereich von 0,05 bis 5 mm sind.

2. Das Verfahren nach Anspruch 1, wobei das Treibmittel ausgewählt ist aus HFA227 oder HFA134a oder einer Kombination dieser beiden in variierenden Anteilen.

3. Das Verfahren nach Anspruch 1, wobei die Perlengröße von 0,1 bis 2 mm beträgt.

4. Das Verfahren nach Anspruch 1, wobei das Verhältnis der Perlengröße zum API von 40:1 oder 20:1 oder 15:1 oder 10:1 oder 5:1 beträgt.

5. Das Verfahren nach Anspruch 1, wobei der API ausgewählt ist aus inhalativen Corticosteroiden (ICS), inhalativen SYK-Inhibitoren (iSYK) und JAK-Inhibitoren (iJAK), lang-wirksamen muskarinischen Wirkstoffen (LAMAs), lang-wirksamen β-Agonisten (LABAs), bifunktionellen Muskarinantagonisten/β-2-Agonisten (MABAs) und kurz-wirksamen β-Agonisten (SABAs), oder Kombinationen davon.

6. Das Verfahren nach Anspruch 5, wobei der API ausgewählt ist aus Mometasonfuroat und Glycopyrrolat (GP).

7. Das Verfahren nach Anspruch 6, wobei der API GP (Glycopyrrolat) ist.

8. Das Verfahren nach Anspruch 1, wobei das Mischen über eine bestimmte Zeitspanne von 5 Minuten bis 300 Minuten erfolgt.

9. Ein Produkt, das durch das Mahlverfahren nach Anspruch 1 erhalten wird.

## Revendications

1. Procédé de broyage comprenant (A) l'ajout d'un principe actif, en présence ou en l'abscence d'excipients et/ou d'additifs, et de billes à un récipient ; (B) sceller le récipient et le remplir d'un milieu propulseur ; et (C) mélanger le récipient avec un dispositif de mixage acoustique, dans lequel les billes sont un milieu de broyage en céramique ayant un spectre granulométrique allant de 0,05 à 5 mm.

2. Procédé selon la revendication 1 dans lequel le milieu propulseur est choisi parmi HFA227 ou HFA134a ou une combinaison des deux dans des proportions variables.

3. Procédé selon la revendication 1, dans lequel la taille des billes est de 0,1 à 2 mm.

4. Procédé selon la revendication 1, dans lequel le rapport entre la taille des billes et le principe actif est de 40 :1, ou 20 :1, ou 15 :1, ou 10 :1 ou 5 :1.

5. Procédé selon la revendication 1 dans lequel le principe actif est choisi parmi des corticostéroïdes inhalés (ICS), des inhibiteurs de SYK (iSYK) et JAK (iJAK) inhalés, des agents muscariniques à action prolongée (LAMA), des β-agonistes à action prolongée (LABA), des antagonistes muscariniques et agonistes β2 à action bifonctionnelle (MABA), et les β-agonistes à action brève (SABA), ou des combinaisons de ceux-ci.

6. Procédé selon la revendication 5, dans lequel le principe actif est choisi parmi le mometasone furoate et le glycopyrrolate (GP).

7. Procédé selon la revendication 6, dans lequel le principe actif est le GP (glycopyrrolate).

8. Procédé selon la revendication 1, dans lequel le mélange a lieu durant une période de temps spécifiée de 5 minutes à 300 minutes.

9. Produit obtenu par le procédé de broyage selon la revendication 1.
